# EUROPEAN PATENT APPLICATION

(11) **EP 2 476 754 A1**
(43) Date of publication of application: **18.07.2012**
(21) Application number: 11151018.6
(22) Date of filing: 14.01.2011
(51) Int. Cl.: C12N 15/67, C12N 15/10, C12N 15/85, C07K 16/46, C12N 15/62

(54) **Methods for the identification and repair of amino acid residues destabilizing single-chain variable fragments (scFv)**

(71) Applicant: Bundesrepublik Deutschland, Letztvertreten Durch Den Präsidenten Des Paul-Ehrlich-Instituts, 63225 Langen (DE)
(72) Inventor: Buchholz, Christian, 60594 Frankfurt (DE); Schneider, Irene, 63456 Hanau (DE)
(74) Representative: Grund, Martin

(57) **Abstract**

The invention relates to a method for identifying stabilizing amino acids in single chain antibody fragments.

SEQUENCE LISTING

<110>  Bundesrepublik Deutschland, letztvertreten durch den
       Präsidenten des Paul-Ehrlich-Instituts Prof. Dr. Klaus Cichutek

<120>  Methods for the identification and repair of amino acid residues
       destabilizing single-chain variable fragments (scFv)

<130>  141-006

<160>  5

<170>  PatentIn version 3.5

<210>  1
<211>  121
<212>  PRT
<213>  Artificial Sequence

<220>
<223>  VH

<400>  1

Gln Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Phe Ile Arg Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Gly Leu Gly Asp Gly Thr Tyr Phe Asp Tyr Trp Gly
            100                 105                 110

Gln Gly Thr Thr Val Thr Val Ser Ser
        115                 120

<210>  2
<211>  111
<212>  PRT
<213>  Artificial Sequence

<220>
<223>  VL

<400>  2

Gln Ser Ala Leu Thr Gln Pro Ala Ser Val Ser Gly Ser Pro Gly Gln
1               5                   10                  15

Ser Ile Thr Ile Ser Cys Ser Gly Ser Ser Ser Asn Ile Gly Asn Asn
            20                  25                  30

Ala Val Asn Trp Tyr Gln Gln Leu Pro Gly Lys Ala Pro Lys Leu Leu
        35                  40                  45

Ile Tyr Tyr Asp Asp Leu Leu Pro Ser Gly Val Ser Asp Arg Phe Ser
    50                  55                  60

Gly Ser Lys Ser Gly Thr Ser Ala Phe Leu Ala Ile Ser Gly Leu Gln
65                  70                  75                  80

Ser Glu Asp Glu Ala Asp Tyr Tyr Cys Ala Ala Trp Asp Asp Ser Leu
                85                  90                  95

Asn Gly Pro Val Phe Gly Gly Gly Thr Lys Leu Thr Val Leu Gly
            100                 105                 110

<210>  3
<211>  123
<212>  PRT
<213>  Artificial Sequence

<220>
<223>  VH

<400>  3

Gln Val Gln Leu Gln Gln Ser Gly Pro Glu Leu Ile Lys Pro Gly Ala
1               5                   10                  15

Ser Val Lys Met Ser Cys Lys Ala Ser Gly Tyr Thr Phe Thr Ser Tyr
            20                  25                  30

Val Met His Trp Val Lys Gln Lys Pro Gly Gln Gly Leu Glu Trp Ile
        35                  40                  45

Gly Tyr Ile Asn Pro Tyr Asn Asp Gly Thr Lys Tyr Asn Glu Lys Phe
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Gly Thr Tyr Tyr Tyr Gly Ser Arg Val Phe Asp Tyr Trp Gly
            100                 105                 110

Gln Gly Thr Thr Leu Thr Val Thr Val Ser Ser
        115                 120

<210>  4
<211>  113
<212>  PRT
<213>  Artificial Sequence

<220>
<223>  VL

<400>  4

Asp Ile Val Met Thr Gln Ala Ala Pro Ser Ile Pro Val Thr Pro Gly
1               5                   10                  15

Glu Ser Val Ser Ile Ser Cys Arg Ser Ser Lys Ser Leu Leu Asn Ser
            20                  25                  30

Asn Gly Asn Thr Tyr Leu Tyr Trp Phe Leu Gln Arg Pro Gly Gln Ser
        35                  40                  45

Pro Gln Leu Leu Ile Tyr Arg Met Ser Asn Leu Ala Ser Gly Val Pro
    50                  55                  60

Asp Arg Phe Ser Gly Ser Gly Ser Gly Thr Ala Phe Thr Leu Arg Ile
65                  70                  75                  80

Ser Arg Val Glu Ala Glu Asp Val Gly Val Tyr Tyr Cys Met Gln His
                85                  90                  95

Leu Glu Tyr Pro Leu Thr Phe Gly Ala Gly Thr Lys Leu Glu Ile Lys
            100                 105                 110

Arg

<210>  5
<211>  246
<212>  PRT
<213>  Artificial Sequence

<220>
<223>  scFv

<400>  5

Gln Val Gln Leu Gln Gln Ser Gly Ala Glu Leu Ala Arg Pro Gly Ala

1               5                   10                  15

Ser Val Lys Met Ser Cys Lys Ala Ser Gly Tyr Thr Phe Thr Thr Tyr
            20                  25                  30

Thr Ile His Trp Val Arg Gln Arg Pro Gly His Asp Leu Glu Trp Ile
        35                  40                  45

Gly Tyr Ile Asn Pro Ser Ser Gly Tyr Ser Asp Tyr Asn Gln Asn Phe
    50                  55                  60

Lys Gly Lys Thr Thr Leu Thr Ala Asp Lys Ser Ser Asn Thr Ala Tyr
65                  70                  75                  80

Met Gln Leu Asn Ser Leu Thr Ser Glu Asp Ser Ala Val Tyr Tyr Cys
                85                  90                  95

Ala Arg Arg Ala Asp Tyr Gly Asn Tyr Glu Tyr Thr Trp Phe Ala Tyr
            100                 105                 110

Trp Gly Gln Gly Thr Thr Val Thr Val Ser Ser Gly Gly Gly Gly Ser
        115                 120                 125

Gly Gly Gly Gly Ser Gly Gly Gly Gly Ser Asp Ile Glu Leu Thr Gln
    130                 135                 140

Ser Pro Lys Phe Met Ser Thr Ser Val Gly Asp Arg Val Asn Val Thr
145                 150                 155                 160

Tyr Lys Ala Ser Gln Asn Val Gly Thr Asn Val Ala Trp Phe Gln Gln
                165                 170                 175

Lys Pro Gly Gln Ser Pro Lys Val Leu Ile Tyr Ser Ala Ser Tyr Arg
            180                 185                 190

Tyr Ser Gly Val Pro Asp Arg Phe Thr Gly Ser Gly Ser Gly Thr Asp
        195                 200                 205

Phe Thr Leu Thr Ile Ser Asn Val Gln Ser Glu Asp Leu Ala Glu Tyr
    210                 215                 220

Phe Cys Gln Gln Tyr His Thr Tyr Pro Leu Thr Phe Gly Gly Gly Thr
225                 230                 235                 240

Lys Leu Glu Ile Lys Arg
                245

## Description

### FIELD OF THE INVENTION

The invention belongs to the field of single chain antibodies and novel methods for improving their stability.

### BACKGROUND OF THE INVENTION

Single chain antibodies (scFv) can be developed that can recognize almost any given target. The procedure for the development of scFv are usually bacteria based phage display systems. The resulting antibodies bind their targets with high affinity. One use of scFvs is the combination of a target specific scFv with a lentiviral vector. For example, the scFv can have a high affinity for a particular tumor antigen or another antigen that is expressed on a target cell. The lentiviral particle can comprise the target specific scFv allowing the lentiviral particle to enter the target cell specifically. The lentiviral particle can then effect genetic change in the target cell, modify the physiology of the target cell or kill the target cell. Usually, the lentiviral particles are produced in mammalian cell culture while the original scFv have been produced in a prokaryotic system. When expressing the hybrid lentiviral particle researches often encounter the problem that the envelope glycoprotein-scFv fusion protein (H-scFv) is not incorporated at all into the lentiviral particle or only with rather low efficiency. It is speculated that the scFvs obtained in a prokaryotic system cannot undergo correct folding in the mammalian cells or are simply instable under the conditions found in mammalian cells during the production of the viral particles. Therefore, many scFv that bind with high efficiency to targets cannot be expressed in mammalian cell culture. In the prior art a variety of models have been developed to overcome these disadvantages. These are usually based on 3D modeling techniques which are complex and time consuming. Other suggestions relate to the incorporation of xenogenic sequences to improve the stability of antigen-recognizing constructs (WO 2008/028601). WO 2005/040344 describes a method involving the generation of stabilized proteins by a combinatorial consensus.

As the stability of a scFv is critical for its applicability in a chimeric H-scFv protein, the inventors developed an in silico method to predict the stability of a given scFv from its primary sequence and to design improved scFv by amino acid (AA) exchange. This method is also part of the invention.

### SUMMARY OF THE INVENTION

The present invention is based on the surprising finding that the application of a novel method allows to identify amino acids in the single-chain variable fragments (scFv) that are highly relevant for the stability of scFv. Therefore, an embodiment relates to a scFv comprising an immunoglobulin heavy chain variable region (VH) and an immunoglobulin light chain kappa variable region (VLK), wherein the VH comprises Ala at position 25, Phe or Tyr at position 29, Ser or Lys at position 86, and Ala or Leu at position 89, the VLK comprises Gln at position 46, Thr at position 87 and Gln at position 97, and wherein the positions are designated by the numbering scheme published by Honegger and Plückthun (JMB (2001) 309:957-670). The AHo (Annemarie Honegger) nomenclature can be applied to any molecule having an antibody-like variable domain so that the CDRs of all these molecules can be aligned at the same position within the numbering scheme. The VLK can further comprise at least one amino acid selected from the group consisting of Asp at position 1, Lys at position 47, Leu at position 55, Ser at position 79, Gly at position 82, and Phe at position 89.

The scFv can be fused to the ectodomain of a hemagglutinin (H) protein, wherein the scFv protein is C-terminally fused to the H protein. Thus the scFv is displayed on the H (H-scFv display).

The scFv can be connected by a linker to the H. The linker is mostly a glycine-serine-linker composed of four consecutive glycine residues and one serine residue, This G₄S linker can be inserted repeatedly, e.g. one, two or three times (G₄S)₃. Other contemplated linkers are the 205Clinker (SSADDAKKDAAKKDDAKKDDAKKDG) or the linker-218 (GSTSGSGKPGSGEGSTKG). Other possible linkers are any flexible or rigid amino acid sequences linking the domains of proteins.

The H-protein can be derived from a paramyxovirus. The virus can be measles virus.

The H protein can be recombinant and/or mutated.

The scFv can be used in the treatment of cancer. An isolated host cell can comprise the scFv. The host cell can be an isolated mammalian cell, for example, a human cell. The host cell can be HEK-293T.

The host cell can be a hybridoma.

An embodiment relates to a method for evaluating the stability of an scFv, comprising the steps:
a) expressing in a mammalian cell line a fusion protein comprising N-terminally a first amino acid sequence comprising a mammalian cellular export signal, and C-terminally a second amino acid sequence comprising the amino acid sequence of an scFv;
b) determming whether the fusion protein is exported from the inside of the cell to the outside of the cell; and optionally
c) designating scFvs comprised in fusion proteins that are exported from the inside of the cell to the outside of the cell as stable, and designating scFvs comprised in fusion proteins that are not exported from the inside of the cell to the outside of the cell as unstable.

The method can be used to optimize the stability of an scFv and further comprise the steps:
d) determining the germline of an scFv analyzing the amino acid sequences of a first group of scFvs designated as stable in step c);
e) identifying in the amino acid sequences of the scFvs analyzed in step d) an amino acid that is located at a given position in the amino acid sequence in at least 75%, 80%, 85%, 90%, or 95% of the amino acid sequences;
f) replacing in an amino acid sequence of an scFv designated as unstable in step c) the amino acid located at the given position in the amino acid sequence with the amino acid identified in step e) to thereby obtain an optimized fusion protein; and
g) determining whether the fusion protein obtained in step f) is exported from the inside of the cell to the outside of the cell,
wherein the steps e) - g) can be repeated.

The first amino acid sequence can correspond to the measles H-protein. The H protein can have the native sequence or a recombinant or mutated sequence.

The mammalian cell line can be HEK-293T.

The outside of the cell includes expression on the surface - of a cell. For example, the H protein is normally transported as an integral membrane protein and a scFv when C-terminally fused to the H protein will be displayed outside the cell.

Detection of the export can be performed by detection of the first amino acid sequence or the scFv or a Tag (e.g. His-Tag) C-terminally of the scFv. Detection can be performed via FACS or immune fluorescence.

The method can comprise determming the amount of fusion protein that is exported from the cell. The method can comprise performing the method on at least one other fusion protein comprising N-terminally a first amino acid sequence comprising a mammalian cellular export signal, and C-terminally a second amino acid sequence comprising the amino acid sequence of an scFv. The first amino acid sequence can be the H-protein, optionally of measles. This other fusion protein can serve as a reference for the amount of fusion protein exported. This other fusion protein can be a fusion protein comprising a scFv obtained from a phage display. This reference fusion protein has not been optimized after being obtained by phage display by the replacement of amino acids, for example, with more stabilizing amino acids. Thus, the reference fusion protein is either not exported to the outside of the cell (fraction of transiently transfected cells positive for cell surface expression of H-scFv < 2%) or only exported at a rate that can be substantially improved by stabilisation.

The fusion protein can generally be part of lentiviral virus particle, i.e. vector. The fusion proteins of the invention, for example, the above described reference fusion protein and the above described fusion proteins can be part of a lentiviral virus particle. Methods for obtaining such particles are described in the detailed part of the description. If a full virus particle comprising the fusion proteins of the invention is produced by the cell the virus particles are released from the packaging cell. Such produced vector particles can be analysed for their content of infectious/transducing units (t.u.), the so called titer. Reference fusion proteins, will generally give titers below 10⁵ t.u./175cm² of vector particle packaging cells (VPC). Virus particles containing fusion proteins comprising optimized scFv as defined below will be produced at higher titers than the virus particles comprising the reference fusion proteins. By screening ~30 scFv sequences from different sources, only half of the fusion proteins were presented at the cell surface, and of those only 7 scFv gave titers above 1x10⁵ tu/175cm² VPC. In those scFv amino acids can be identified that confer increased stability to the modified/optimized scFv and the virus particles containing said scFvs. Increased stability can be defined as provided a higher cell surface expression level of wherein the ratio between the amount of a fusion protein comprising the modified scFv compared to reference fusion protein, can be 1.05, 1.10, 1.15, 1.20, 1.30, 1.40, 1.50, 1.60, 1.70, 1.80, 1.90, 2.00, 3.00, 4.00, 5.00, 6.00, 7.00, 8.00, 9.00, 10.00, 50.00, 100.00, 200.00, 300.00, 400.00, 500.00, 600.00, 700.00, 800.00, 900.00, or 1000.00 or above any of these values. The amount can be determined by FACS or Western Blot. If the reference fusion protein is not expressed at the cell surface at all, an optimized fusion protein is defined as a fusion protein that exhibits a surface expression of at least 5%.

An embodiment relates to a method for optimizing the expression of a single chain variable fragment (scFv) comprising the steps of:
a. expressing in mammalian cells a set of fusion proteins wherein each amino acid sequence of the fusion proteins comprises N-terminally a first amino acid sequence comprising a mammalian cellular export signal and C-terminally a second amino sequence comprising the amino acid of an scFv
b. determining a first group of fusion proteins wherein the fusion proteins of the first group are exported from the inside of the cell to the outside of the cell and a second group of fusion proteins wherein the fusion proteins of the second group are not exported from the inside of the cell to the outside of the cell
c. aligning the amino acid sequences of the scFvs of the fusion proteins of the first group determined in step b
d. identifying at least one amino acid that is present in at least 75% of the amino acid sequences of the scFvs of the first group
e. replacing in an amino sequence of the second group at least one amino acid which is at the same position as the amino acid identified in step d with said amino acid identified in step d. The amino acid comprising a mammalian cellular export signal can be the ectodomain of the hemagglutinin protein of the measles virus (H protein).

The cells can comprise the nucleic acid sequences required for the production of lentiviral particles.

The first group of fusion proteins can be characterized by being associated with a titre of at least 10⁵ transducing units (t.u.)/175 cm² vector producing cells.

Step b can further comprise the step of determining the germlines of the scFv in the first group of fusion proteins thereby determining corresponding germline subgroups and
ii. in step c the amino acid sequences of the scFvs of each respective germline can be aligned
iii. And in step 1.d. the at least one amino acid that is present in at least 75% of the amino acid sequences can be identified per subgroup.

In step 1.c the aligned amino acid sequence can be the variable heavy chain (VH) or the variable light chain (VL), wherein VL can be VL (kappa) or VL (lambda).

The germlines can be murine (mouse or rat), rabbit or human or any other spezies. The Vh and VL of a scFv can be derived from any germline gene of a species.

The amino acids identified in step 1.d. in the VL (kappa) sequence can be selected from the group consisting of 1 Asp, 46 Gln, 47 Lys, 55 Leu, 79 Ser, 82 Gly, 87 Thr, 89 Phe, and 97 Gln. The amino acids identified in step 1.d. in the VH can be selected from the group consisting of 25 Ala, 30 Phe, 30 Tyr, 86 Ser, 86 Lys, 89 Ala, and 89 Leu.

The method can further comprise a step e relating to the expression of the nucleic acid obtained in step 1.d in a mammalian cell line.

The mammalian cells can be cells from the HEK-293T cell line.

In the fusion protein the amino terminus of the scFv can be fused to the carboxyl terminus of the H-protein.

An embodiment relates to a method for evaluating the stability of an scFv comprising the following steps:
a. expressing in mammalian cells a fusion protein wherein each amino acid sequence of the fusion proteins comprises N-terminally a first amino acid sequence comprising a mammalian cellular export signal and C-terminally a second amino sequence comprising the amino acid of an scFv
b. determining whether the fusion protein is exported from the inside of the cell to the outside of the cell
c. designating scFvs in exported fusion constructs as not being unstable scFvs.

The mammalian cell line can be a human cell line, optionally the HEK-293T cell line.

An embodiment relates to an optimized scFv selected from the group consisting of scFv anti-CD30, anti-CD19 (clone 4G7), anti-CD8 (Okt8), anti-GluR-D (Fab22), anti-NK-cells (KYK) and anti-mCD105 (mE12).

Additional embodiments of the present invention are described in the claims and the detailed description of the invention.

### Definitions

"**vector particles**", as used in the present invention, refer to replication deficient vectors. Thus, the lentiviral vector particles of the present invention are lentiviral vectors that contain an RNA which, upon infection of a host or target cell, is reverse-transcribed and inserted into the genome of said cell. However, these vector particles only contain an incomplete genome of the lentivirus from which they are derived. In general, the RNA molecule of the vector particle does not comprise the genetic information of the *gag*, *env*, or *pol* genes itself, which is a known minimal requirement for successful replication of a retrovirus. As a result, the vector particles are replication deficient, i.e. they are not able to transduce a host or target cell with the genetic information required for their own replication.

A vector particle thus comprises a minimum of the Gag, Pol, and Env proteins and an RNA molecule (which can be an expression vector). The Gag, Pol, and Env proteins needed to assemble the vector particle are derived from the retrovirus and are provided *in trans* by means of a packaging cell line, for example HEK-293T. The RNA molecule is derived from the genome of the retrovirus but does not comprise at least one of the *gag*, *env*, or *pol* genes itself. However, to produce a safe and efficient vector particle, in general all three of said genes are lacking, as well as any other nonessential genes. In case of HIV-1, such unnecessary genes of the HIV-1 genome that can be deleted from the RNA molecule include *tat*, *vif*, *vpr*, *vpu* and *nef.*

The RNA molecule still comprises all elements, for example, the psi element and LTRs, of the retroviral genome which are required for an effective packaging of the RNA into the resulting vector particles. In the case of an expression vector, the RNA molecule preferably comprises a further gene (usually heterologous) that is under the control of a suitable promoter, for example, the CMV promoter, and is thus expressed upon integration of the gene into the genome of the host or target cell.

Therefore, one example of an RNA molecule that may be used to generate a retroviral vector particle is based on the HIV-1 genome and comprises the LTRs, the psi element and the CMV promoter followed by the gene to be transduced, for example, the gene for a GFP protein. The *gag*, *env*, *pol*, *tat*, *vif*, *vpr*, *vpu* and *nef* genes of HIV-1 are removed or expression of their gene products is prevented by, for example, frame shift mutation(s). The minimal requirements for a lentivirus vector based on HIV-1 have been described by Kim et al. (J. Virology, 72:811-816, 1998).

The above-described RNA molecule together with the *gag*, *pol* and *env* proteins, provided *in trans* by the packaging cell line, are then assembled into the vector particles or vector particles, which will be able to efficiently infect their target or host cells, reverse-transcribe the RNA molecule that may comprise a heterologous gene under the control of the CMV promoter, and integrate said genetic information into the genome of the target/host cells. However, as the genetic information for the *gag*, *pol* and *env* proteins is not present on the transduced RNA molecule, the vector particles or vector particles will be replication deficient, i.e. no new generation of said vector particles will thus be generated by the transduced cell.

The term "**pseudotyped**", "**pseudotyped vector**" or "**pseudotyped vector particle**", as used in the present invention, refers to a vector particle bearing envelope glycoproteins derived from other viruses having envelopes. The host range of the lentiviral vectors vector particles of the present invention can thus be expanded or altered depending on the type of cell surface receptor used by the glycoprotein.

As explained in the foregoing section, the *gag*, *pol* and *env* proteins needed to assemble the vector particle are provided *in trans* by means of a packaging cell line, for example, HEK-293T. This is usually accomplished by transfection of the packaging cell line with one or more plasmids containing the *gag*, *pol* and *env* genes. For the generation of pseudotyped vectors, the *env* gene, sourceally derived from the same retrovirus as the *gag* and *pol* genes and as the RNA molecule or expression vector, is exchanged for the envelope protein(s) of a different enveloped virus. As an example, the F and H protein of measles virus (MeV) are used.

Thus, an exemplary pseudotyped vector particle based on the HIV-1 retrovirus comprises the (1) HIV-1 Gag and Pol proteins, (2) an RNA molecule derived from the HIV-1 genome that may be used to generate a retroviral vector particle based on the HIV-1 genome lacking the *gag*, *env*, *pol*, *tat*, *vif*, *vpr*, *vpu* and *nef* genes, but still comprising the LTRs, the psi element and a CMV promoter followed by the gene to be transduced, for example, a gene for the GFP protein, and (3) the F and H proteins of MeV_{Edm}.

The resulting pseudotype vector particle will thus display the tropism of MeV_{Edm}, i.e. have the ability to effectively transduce cells expressing the CD46 and/or SLAM proteins on their surface.

"**Cell entry targeted**" or "**targeted**" lentiviral vector particles are pseudotyped vector particles of the present invention, wherein the H protein is a chimeric protein that does not interact with CD46 or SLAM and further has a single chain antibody at its ectodomain. Thus, the host range of a cell entry targeted lentiviral vector particles of the present invention is not expanded or altered depending on the tropism of the virus the H protein is derived from, but, depending on the specificity of the single chain antibody fused to the H protein.

"**Functional scFv**" or "**stable scFv**" describes scFv which when fused to the ectodomain of the H-protein are presented at the surface of cells transfected with the plasmid coding for the H-scFv protein and allow production - by having the H-scFv protein incorporated - of targeted lentiviral vector particles with a titre > 10⁵ t.u./175cm² vector producing cells (VPC).

"**Non-functional scFv**" or "**unstable scFv**" describes scFv which when fused to the ectodomain of the H-protein are not presented at the surface of cells transfected with the plasmid encoding the H-scFv protein and thus do not give rise to targeted lentiviral vectors.

"**Partially unstable scFv**" describes scFv which when fused to the ectodomain of the H-protein are presented at the surface of cells transfected with the plasmid coding for the H-scFv protein but do not allow production - although having the H-scFv protein incorporated - of targeted lentiviral vector particles with a titre > 10⁵t.u./175cm² VPC.

A pseudotyped vector particle "**derived from**", for example, HIV-1, as used in the present invention, refers to a vector particle in which the genetic information for the RNA and/or the Gag and Pol proteins comprised by the vector particle sourceally stems from said retrovirus, in the above case, HIV-1. As described above, the sourceal retroviral genome can comprise mutations, such as deletions, frame shift mutations and insertions.

The term "**cytoplasmic portion**", "**cytoplasmic tail**" or "**cytoplasmic region**", as used in the present invention, refers to the portion of the respective protein that is adjacent to the transmembrane domain of the protein and, if the protein is inserted into the membrane under physiological conditions, extends into the cytoplasm. For the MeV H protein, the domain is identified by the amino acid sequence "PYVL". The cytoplasmic portion of the MeV H protein typically consists of the 34 N-terminal amino acids, in the case of MeV_{Edm}, the protein consists of the amino acid sequence "MSPQRDRINAFYKDNPHPKGSRIVINREHLMIDR".

The person skilled in the art will readily be able to introduce mutations as, for example, additions and deletions, into a given nucleic acid or amino acid sequence. Such known methodologies are, for example, disclosed in Sambrook *et al.* (1989).

Using the nucleic acid sequences disclosed herein, the skilled person can further design nucleic acid structures having particularly desired functions in various types of applications. For example, the artisan can construct oligonucleotides or polynucleotides for use as primers in nucleic acid amplification procedures, such as the polymerase chain reaction (PCR), ligase chain reaction (LCR), Repair Chain Reaction (RCR), PCR oligonucleotide ligation assay (PCR-OLA), and the like. Oligonucleotides useful as probes in hybridization studies, such as *in situ* hybridization, can also be constructed. Numerous methods for labeling such probes with radioisotopes, fluorescent tags, enzymes, and binding moieties (e.g., biotin) are known, thus the probes of the present invention can be adapted for detectability in a straightforward manner.

Oligonucleotides can also be designed and manufactured for other purposes. For example, the present invention enables the artisan to design antisense oligonucleotides and triplex-forming oligonucleotides for use in the study of structure/function relationships. Homologous recombination can be implemented by adapting the nucleic acid of the invention for use as a targeting means.

The invention particularly includes nucleic acids sequences or homologs thereof, or unique fragments thereof. In the present invention, the sequence of a nucleic acid molecule that encodes a resulting protein is considered homologous to a second nucleic acid molecule if the nucleotide sequence of the first nucleic acid molecule is at least about 70% homologous, preferably at least about 80% homologous, and more preferably at least about 85%, 90%, 95% or 99% homologous to the sequence of the second nucleic acid molecule. Homology between two nucleic acid sequences may be readily determined using the known BLASTN algorithm (Altschul, *et al*., 1990) with default settings. As a further example, another known test for ascertaining the homology of two nucleic acid sequences is whether they hybridize under normal hybridization conditions, preferably under stringent hybridization conditions.

The protein encoded by the nucleic acid of the present invention further includes functional homologs. A protein is considered a functional homolog of another protein for a particular function, if the homolog has a similar function as the sourceal protein. The homolog can be, for example, a fragment of the protein, or a substitution, addition, or deletion mutant of the protein.

Determining whether two amino acid sequences are substantially homologous is typically based on FASTA searches in accordance with Pearson & Lipman (1988). For example, the amino acid sequence of a first protein is considered to be homologous to that of a second protein if the amino acid sequence of the first protein shares at least about 70% amino acid sequence identity, preferably at least about 80% identity, and more preferably at least about 85%, 90%, 95% or 99% identity, with the sequence of the second protein.

"**Psi positive**" and "**psi negative**", as used in the present application, refers to a nucleic acid molecule where the retroviral psi element is present and absent, respectively. The psi element is a cis-acting signal located near the 5' end of the retroviral genome and designates a packaging signal, which is of importance during assembly of the viruses and leads to the incorporation of the viral RNA into the viral core. Thus, a psi negative RNA does not comprise the retroviral psi element and consequently will not be assembled into a vector particle of the present invention; in contrast, a psi positive RNA that does comprise said psi element will be effectively assembled into the vector particle.

"**Titers**" and "**transduction efficiency**" refer to the ability of a vector particle to enter a cell and integrate the genetic information, in particular RNA information, into the genome of the cell. Thus, the terms "titer" and "transduction efficiency" can be used synonymously. The titer/transduction efficiency can, for example, be determined by incorporation of a gene coding for a detectable marker, for example, under the control of a CMV promoter, into the expression vector of the vector particle. Upon transduction of such a detectable marker by means of the vector particle, the transduced cells will then express the detectable marker. If the detectable marker is, for example, the GFP protein, the number of cells infected by a given number of vector particles can readily be determined, e.g. by FACS analysis, and directly corresponds to the titer or transduction efficiency of the vector particles that have been used for transduction. The titer or transduction efficiency of a given vector particle thus refers to the number of cells transduced relative to the number of vector particles used.

The titer or transduction efficiency is used as a means to characterize and compare vector particles with regard to their ability to transduce their target cells. Thus, a vector particle having an "increased titer" or an "increased transduction efficiency" is able to transduce a higher number of cells at a given vector particle concentration than a different vector particle having the same vector particle concentration.

The term "**cell marker**", or "**cell surface marker**", as used in the present invention, refers to a molecule present on the surface of a cell. Such molecules can be, *inter alia*, peptides or proteins that may comprise sugar chains or lipids, antigens, clusters of differentiation (CDs), antigens, antibodies or receptors. Since not all populations of cells express the same cell markers, a cell marker can thus be used to identify, select or isolate a given population of cells expressing a specific cell marker. As an example, CD4 is a cell marker expressed by T helper cells, regulatory T cells, and dendritic cells. Thus, T helper cells, regulatory T cells, and dendritic cells can be identified, selected or otherwise isolated, *inter alia* by a FACS cell sorter, by means of the CD4 cell marker.

The term "**export signal**" here refers to an N-terminal amino acid sequence that leads to the transport of a protein comprising said signal to the surface of a cell and/or export of the protein to the outside of the cell. A variety of export signal and amino acids sequences or proteins containing such signal is known in the art. A protein comprising an export signal is the hemagglutinin protein (H-protein), in particular, the H-protein of measles virus.

The term "**selectable marker**" or "**selectable marker gene**", as used in the present invention, refers to a gene and its corresponding product that allow detection, selection and/or isolation of said product. If such a selectable marker is expressed by a cell, consequently such a cell or a population of such cells can be detected, selected and/or isolated.

Selectable markers can be, *inter alia,* a molecule, preferably a peptide or protein, detectable by fluorescence, an enzyme catalyzing a reaction of which the resulting product is monitored, a molecule that confers a resistance to, *inter alia*, an antibiotic or another toxic substance, or a molecule interacting with a non-toxic substance to render it toxic. Detection, selection and/or isolation can be carried out, *inter alia,* by FACS, a FACS cell sorter, use of fluorescence microscopy, or by the use of antibiotics or cytotoxic substances, use of precursors of cytotoxic substances.

### BRIEF DESCRIPTION OF THE FIGURES

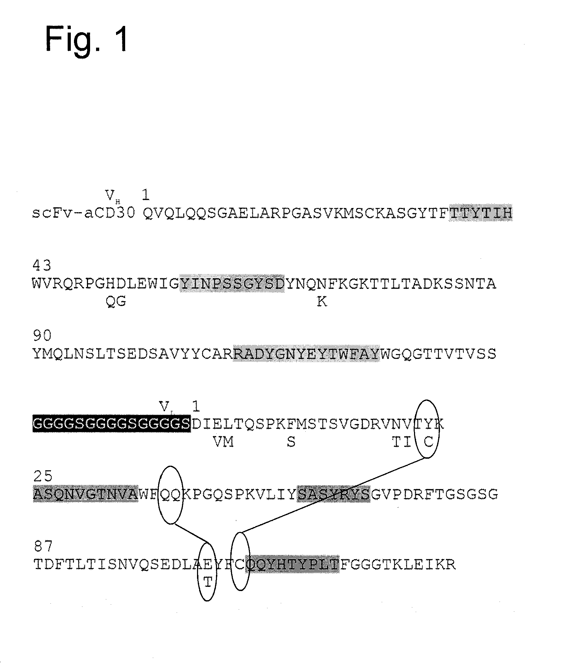

Fig. 1: AS-sequence of the scFv-antiCD30 and the changed AS (lower sequence lane) in the corrected -scFv-antiCD30corr
   Critical AS in the scFv framework sequence are substituted by the underneath AS. The important Tyrosin-Glutamm bridge (T-Q) and Disulfid bridge (C-C) are specified. Variable CDRs of the VH (light gray) and the VL(dark gray) and the linker between VH and VL (black) are indicated
Fig. 2: Influence of some corrections on the intracellular localisation of H-CD30 variants
   293T cells were transfected with the H-CD30 variants comprising different corrections. The H-protein was stained the K82-anti-H antibody and a Cy3-labelled secondary anti-mouse IgG antibody. Immune fluorescence microscopy revealed that the different mutations influenced the ability of the protein to reach the cell surface, a prerequisite for lentivector (LV) production. (white arrows: intracellular staining of retained protein, gray arrows: cell surface staining of correct transported protein)

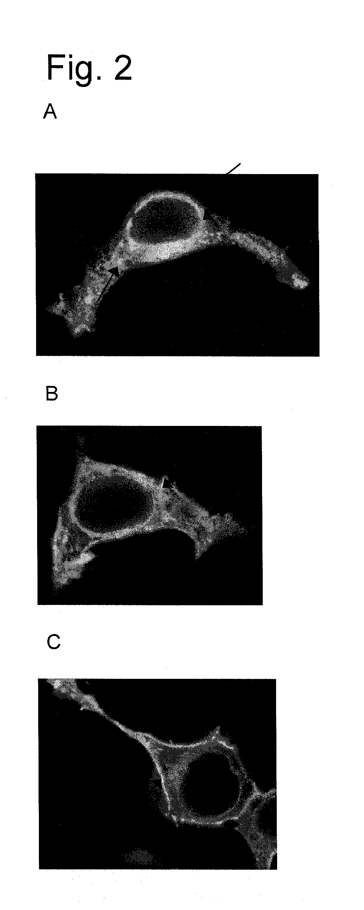

Fig. 2 A: restabhshed T-Q bridge
Fig. 2 B: restablished C-C and T-Q bridge
Fig. 2 C: restablished C-C and T-Q bridge, and HD/QG exchange

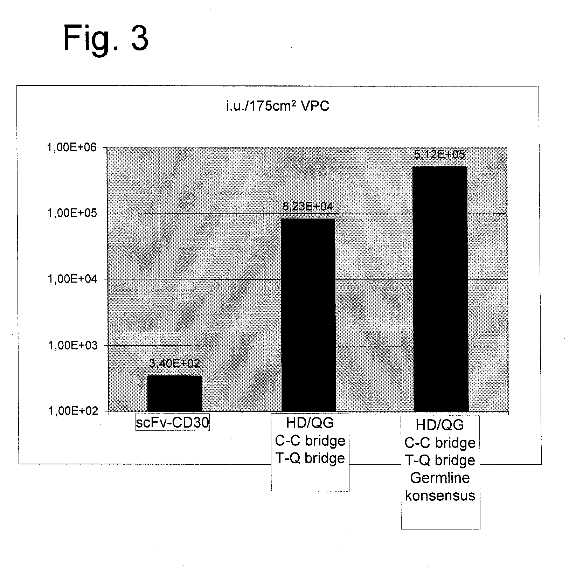

Fig. 3: Influence of the different corrections on the titer of the according CD30-LV 293T cells were transfected with the LV components and different H-CD30 variants. The cell surface presentation of the H-CD30 protein strongly influences the LV titers that can be produced with the according variant.

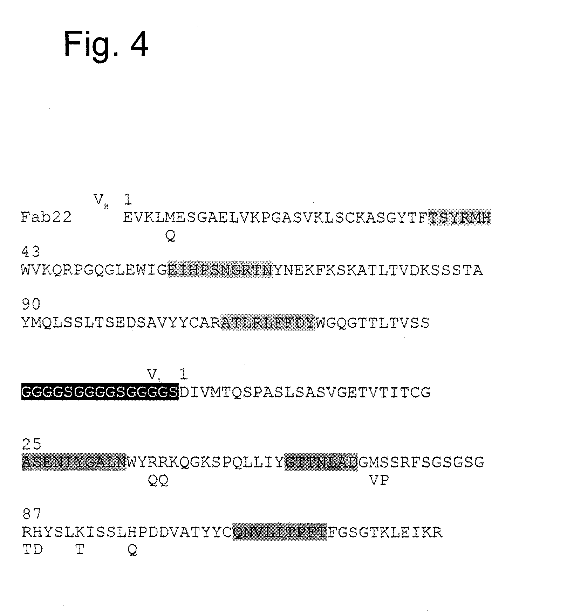

Fig. 4: AS-sequence of the scFv-antiGluRD (Fab22) and the changed AS (lower sequence lane) in the corrected scFv-antiGluRD (Fab22_{corr})
   Critical AS in the scFv framework sequence are substituted by the underneath AS. Variable CDRs of the V_{H} (light gray) and the V_{L}(dark gray) and the linker between V_{H} and V_{L} (black) are indicated.

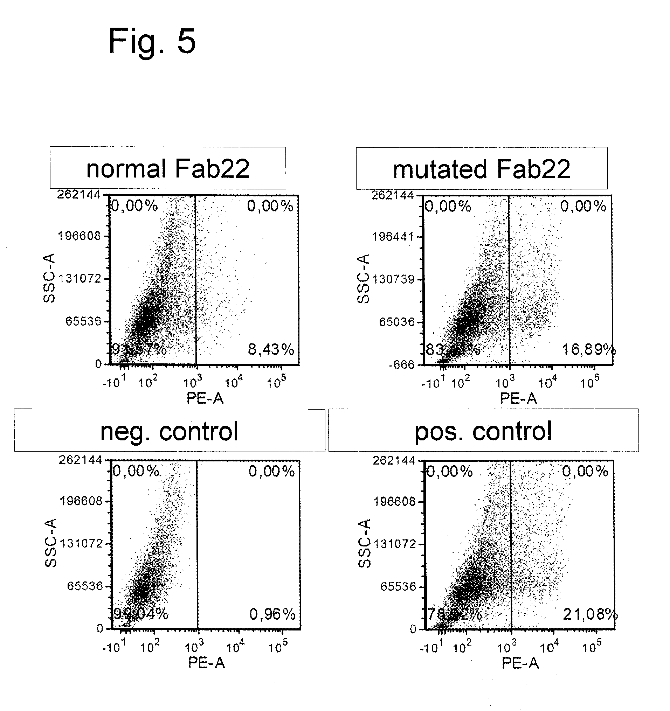

Fig. 5: Surface expression of H-Fab22 293T cells were transfected with the different H-Okt8 variants and after staining ot the His-Tag at the ectodomain of the scFv with a PE-labelled anti His antibody cells were analysed by FACS.

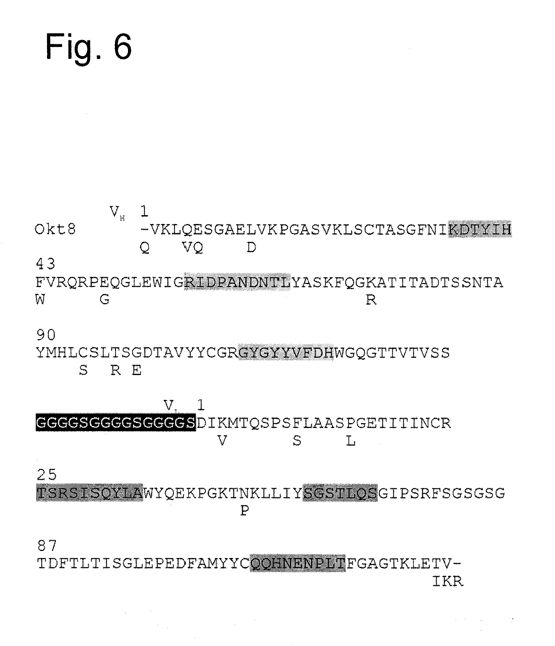

Fig. 6: AS-sequence of the scFv-antiCD8 (Okt8) and the changed AS (lower sequence lane) in the corrected scFv-antiCD8 (Okt8_{corr}). Critical AS in the scFv framework sequence are substituted by the underneath AS. Variable CDRs of the V_{H} (light gray) and the V_{L}(dark gray) and the linker between V_{H} and V_{L} (black) are indicated.

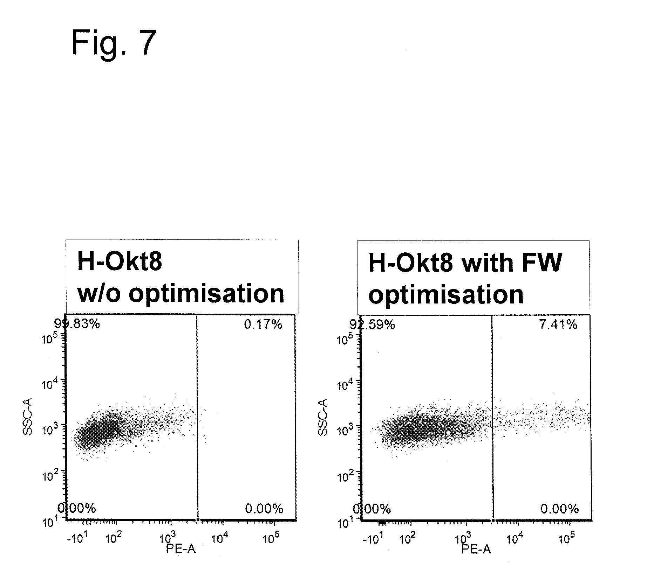

Fig. 7: Surface expression of H-Okt8. 293T cells were transfected with the different H-Okt8 variants and after staining of the His-Tag at the Ectodomain of the scFv with a PE-labelled anti His antibody cells were analysed by FACS

### DETAILED DESCRIPTION OF THE INVENTION

### I. Overview: Retroviridae and generation of lentiviral vector particles

### 1. Structural and Genetic Features of Lentiviral Genomes

The genome of known replication-competent retroviruses comprises the four coding domains (*gag*, *pro*, *pol*, *env*):

The first of the four coding domains (*gag*) encodes a polypeptide (Gag) whose cleavage products are the major structural proteins of the virus core and consist of: Matrix (MA), which is associated with the inside of the virus envelope; capsid (CA), a principal structural protein of the virion core; and nucleocapsid (NC), a small, basic protein which is tightly bound to the viral genomic RNA and forming a ribonucleoprotein complex within the core.

The second of the four coding domains (*pro*) encodes part of the polyprotein (Gag-Pro or Gag-Pro-Pol) whose cleavage products typically include protease (PR) and occasionally dUTPase (DU).

The third of the four coding domains (*pol*) encodes part of the polyprotein (Gag-Pro-Pol), whose cleavage products typically include reverse transcriptase (RT) and integrase (IN) and, in some lentiviruses, dUTPase (DU). In spumaviruses, *pol* is expressed via a spliced mRNA as Pol-Pol polyprotein.

Finally, *env* encodes a polyprotein (Env) whose cleavage products SU (surface) and TM (transmembrane) comprise the structural proteins of the viral envelope. These proteins are not required for the assembly of enveloped viral vector particles, but they do have an essential role in the cellular entry process. The SU domain binds to a specific receptor molecule on the target cell. This binding event appears to activate the membrane fusion-inducing potential of the TM protein and, by a process that remains largely undefined, the viral and cell membranes then fuse. The specificity of the SU/receptor interaction defines the host range and tissue tropism of a retrovirus. As a consequence, viral vector particles lacking envelope glycoproteins are noninfectious, and cells lacking a receptor are nonpermissive for viral entry. Viruses may bind weakly to resistant cells through relatively nonspecific interactions, but, in the absence of a specific receptor molecule, they are unable to initiate the infection process.

In addition, retroviruses contain two long terminal repeats (LTRs), a region of several hundred (~300-1800) base pairs composed of U3-R-U5 (5'to 3') which are located at both ends of the unintegrated and integrated (proviral) genome.

Furthermore, a psi element is present within the retroviral genome. The psi element is a cis-acting signal, located near the 5' end of the genome and designates a packaging signal, which is of importance during virus assembly and leads to the incorporation of the viral RNA into the viral core.

More complex retroviruses typically comprise additional regulatory genes. In case of HIV, these include *tat*, *rev*, *vif*, *nef*, *vpu* und *vpr*.

### 2. Lentiviral Vector Particles and their Generation

Lentiviral vector particles are replication deficient lentiviral vectors and are useful for transducing a nucleic acid sequence into a target/host genome. Such vector particles comprise a minimum of the Gag, Pol and Env proteins and an RNA molecule, which can be an expression vector. This RNA molecule or expression vector is usually derived from the genome of the sourceal retrovirus, which means that it comprises all the necessary elements for an effective packaging into the resulting lentiviral vector particles (*inter alia* the psi element and LTRs). However, in order for the lentiviral vector particle to be replication deficient, the RNA molecule does not comprise the genetic information of the *gag*, *env*, or *pol* genes itself. Instead, a normally heterologous nucleic acid sequence to be integrated into the target/host genome is present in the expression vector. The minimal requirements for a lentivirus vector based on HIV-1 have been described by, for example, Kim et al. (J. Virology, 72:811-816, 1998).

Thus, for generating lentiviral vectors, three basic components, usually provided on separate plasmids, are required: a psi-negative *gag*/*pol* gene, a psi-negative *env* gene and a psi-positive expression vector. Co-transfection of these components into a packaging cell line, i.e. a suitable eukaryotic cell line, will lead to the expression of the Gag, Pol and Env proteins as well as generation of psi-positive RNA (however, use of cell lines that are stably transfected with one or more of these basic components is certainly possible). Since the *gag*/*pol* and *env* genes are psi-negative, the mRNA generated prior to expression of the corresponding proteins will not be assembled into the lentiviral vector particles. Contrasting this, the psi-positive RNA transcribed from the expression vector will be included in the resulting lentiviral vector particle.

Consequently, replication deficient lentiviral vector particles will be generated comprising the Gag, Pol and Env proteins provided *in trans* by the packaging cell line, as well as the psi-positive RNA transcript of the expression plasmid lacking the genetic information for autonomous replication. However, as the lentiviral vector particles comprise the Gag, Pol and Env proteins, they will be able to efficiently infect their target/host cells, reverse-transcribe their RNA, and integrate said genetic information into the genome of the target/host.

### 3. Pseudotyped Heterologous Lentiviral Vector Particles

The host range of lentiviral vectors can be expanded or altered by a process known as pseudotyping. The process of pseudotyping a lentivirus or a lentiviral vector particle is generally understood to result in a lentiviral vector particle that consists of a lentiviral vector particle bearing glycoproteins derived from other enveloped viruses. Such vector particles possess the tropism of the virus from which the glycoprotein(s) were derived.

As described above, for generating lentiviral vector particles, three basic components, usually provided on separate plasmids, are required. Within a packaging cell line, a psi-negative *gag*/*pol* gene of the sourceal lentivirus, a psi-negative *env* gene of the sourceal lentivirus, and a psi-positive expression vector are needed. For pseudotyped lentiviral vector particles, the *env* gene of the sourceal retrovirus is replaced by one or more envelope gene(s) of another enveloped virus, for example, the genes coding for the F and H proteins of MeV. Furthermore, the RNA transcribed from the psi-positive expression vector will be assembled into the resulting lentiviral vector particle. This psi-positive expression vector is usually derived from the genome of the sourceal retrovirus, which means that it comprises all the necessary elements for an effective packaging into to resulting pseudotyped lentiviral vector particles. Furthermore, this expression vector may carry at least one gene which is to be transduced, i.e. integrated into the genome of the target cell and ultimately expressed by said cell.

Several of the proteins required to assemble pseudotyped lentiviral vector particles are detrimental to mammalian cells when they are overexpressed. Thus, for the stable production of pseudotyped lentiviral vectors particles, 293-based cell lines allowing conditional production of virus-encoded proteins using tetracycline regulated promoters were generated (for example see Xu *et al*., 2001). In one embodiment, the packaging cell used for producing the pseudotyped lentiviral vector particles of the present invention is HEK-293T.

The "psi" element referred to above designates a packaging signal, which leads to the incorporation (the "packaging") of the RNA transcribed from the expression vector into the vector particles during the assembly of the vector particles. Thus, "psi" designates the retroviral packaging signal that efficiently controls the packaging of the messenger RNA of the expression vector. In order for packaging to occur, the expression vector also has to be flanked by specific long terminal repeat sequences (LTRs), so the transcription of the RNA of the expression vector into DNA and subsequent integration into the genome of the target cell can successfully be accomplished.

Consequently, during assembly of the pseudotyped lentiviral vector particles, neither the RNA of the *gag*/*pol* gene nor that of the envelope gene encoding, for example, the MeV F and H proteins is incorporated into the lentiviral vector particles. These genes are only transcribed into RNA by the transcription machinery of the packaging cell and further translated into functional proteins. Contrasting this, the psi-positive expression vector is translated into the corresponding RNA and, due to the presence of the psi element and the LTRs, will be incorporated into the resulting lentiviral vector particles and ultimately transduced into the genome of the host cell.

### II. The Lentiviral Vector Particles of the Present Invention

### 1. Lentiviral Vector Particles

The lentiviral vector particles of the present invention are generally produced based on psi-positive lentiviral expression vectors.

Even though any lentiviral expression vector may be suitably be used according to the present invention, the lentiviral expression vector used for the production of lentiviral vector particles is preferably based on a vector selected from the group consisting of HIV-1, HIV-2, SIVmac, SIVpbj, SIVagm, FIV and EIAV (equine infectious anemia virus). It will be obvious for the skilled person that these lentiviral expression vectors need not comprise the complete genomic information of the respective lentivirus; rather these lentiviral vectors only need to comprise the necessary elements for effective packaging into the resulting lentiviral vector particles (see above).

In a further embodiment, the pseudotyped lentiviral vector particle of the invention is derived from a lentivirus, preferably derived from a lentivirus selected from the group consisting of HIV-1, HIV-2, SIVmac, SIVpbj, SIVagm, FIV and EIAV, and even more preferably, is derived from HIV-1.

### 2. Pseudotyped Heterologous Lentiviral vector particles

For generating the lentiviral vector particles of the present invention, the *env* gene of the sourceal lentivirus is exchanged with the truncated F and H proteins of a morbillivirus, or MeV, which are co-expressed in the packaging cell.

In the following, properties of the *Morbilliviridae* are described and exemplified using the measles virus (MeV). However, it will be clear to the skilled person that the properties, exemplified for MeV, also generally apply for the *Morbiliviridae.*

As described above, the MeV envelope comprises hemagglutinin (H) and fusion (F) glycoproteins.

### 3. Cell Entry Targeted Lentiviral Vector particles

The findings of the present invention are also important in generating the targeted lentiviral vector particles having improved selective cell entry over other known vector particles, wherein the derived truncated H protein is a mutated (Hmut) and chimeric protein, further displaying a single chain antibody, a growth factor, or a ligand to a cell surface marker at its ectodomain. Thus, the C-terminus of the truncated H protein is fused to a single chain antibody.

In a series of targeting experiments, it was found that presently disclosed lentiviral vector particles pseudotyped with a truncated F protein and a fusion construct of a truncated H protein displaying a single chain antibody at its ectodomain, only transduced cells that expressed the respective cell surface marker to the single chain antibody. The vector particles thus enter those cells expressing the corresponding marker protein; however, the titers are significantly reduced on cells not expressing these markers. Therefore, cell entry and transduction using the lentiviral vector particles of the present invention was demonstrated to be an efficient and effective means for highly selective gene transfer into specific cells. Thus, in a further embodiment the lentiviral vector particles of the present invention can be used to efficiently and effectively transduce cells. In a preferred embodiment such cells are selected from the group consisting of stem cells, cancerous cells, neural cells, EPC, and CD34+ cells.

In another embodiment, the psi-negative expression vector encoding the H protein encodes a H protein that is a chimeric protein which does not interact with CD46 or SLAM and further has a single chain antibody to a cell surface marker at its ectodomain.

In another preferred embodiment, the single chain antibody or ligand is directed against or binding to a cluster of differentiation (CD) marker, a tumor antigen exposed on the cell surface, a tyrosine kinase receptor, a chemokine receptor, a G-protein-coupled receptors, an olfactory receptor, a viral proteins exposed on the surface of chronically infected cells, a neurotransmitter receptor, a stem cell factor, a growth factor, a nerve growth factor, an epidermal growth factor, a vascular endothelial growth factor, a hepatocyte growth factor, an interleukine receptor and/or a cytokine receptor. Even more preferably, the single chain antibody or ligand is directed against or binding a molecule selected from the group consisting of CD4, CD8, CD34, CD20, CD19, CD33, CD133, EGF-R and VEGFR-2, mucin-1, the dopamine receptor, the acetylcholine receptor, the GABA receptor, EGF-R, VEGFR-2, the HIV gp120 protein, (HGF) and erythropoietin.

In another preferred embodiment, the psi-positive lentiviral expression vector comprises at least one gene selected from the group consisting of a selectable marker gene, a gene encoding a fluorescent protein, a gene encoding an antibiotic resistance gene, a gene encoding siRNA, a gene encoding shRNA, a gene encoding an angiogenic factor, a gene encoding an apoptotic factor, a gene encoding a cytotoxic factor, a gene encoding an anti-apoptotic factor, a gene encoding a neuro-protective factor, a gene encoding a viral or bacterial antigen, a gene encoding an anti-viral protein, a gene encoding a tumoral antigen, a gene encoding an immune-stimulatory factor, and a functional copy of a defective or mutated gene in a patient suffering from an inherited disease. Preferably, the gene is selected from the group consisting of GFP, eGFP, apoptosis-inducing protein or a gene coding for a cytotoxic protein, including as, *inter alia,* the TNF-α gene, the p53 gene, an interfering RNA, an interferon gene, and a gene coding for an immune stimulatory protein.

In another preferred embodiment, the packaging cell line stably expresses one or more of the genes encoded by psi-negative lentiviral gag/pol gene, psi-positive lentiviral expression vector or one or two psi-negative expression vector(s) encoding for truncated or complete measles virus H and F proteins.

As source for scFv there are several possibilities: e.g. cloning from hybridoma, selection from phage- or other display libraries and selection from H-display libraries. ScFv are composed of the variable CDR regions and the framework which is highly conserved within a certain germline. Known germlines are Human V_{Lk} 1 - Vₖ7; murine V_{Lk}1-17, Hu V_{L lambda} 1-11; murine V_{L lambda} 1-3, human V_{H} 1-7 murine V_{H} 1-15 Among all germlines certain amino acid residues in the framework regions are highly conserved. Furthermore, certain germlines encode more stable scFv than others (Ewert et al.; J Mol Biol, 2003). Several groups are working on the stabilisation of scFv for prolonged half life, more efficient folding or enhanced thermo/chemostability (all together called here stability). Most approaches for the optimisation of scFv rely on structural comparison of a new sequence with a database (e.g. structure or sequence). In open access databases the information about the contribution of a certain AA to the stability of the scFv coded by a certain germline or about the scFv stability at all is lacking. As we display the scFv fused to the ectodomain of the H-protein, efficient folding and structural stability of the scFv are necessary for the transport of the H-scFv fusion protein to the cell surface. It is important to mention that H protein is a type-2 transmembrane protein and scFv are fused to its carboxyterminus. Thus, during translation, the scFv will only be build after H protein has been translated and started to fold. By measuring the surface expression of the H-scFv fusion protein we are able to test for the stability and proper folding of the scFv, as unfolded proteins are not transported to the cell surface.

Considering the information about surface expression and titre of produced targeted lentiviral vector particles we perform the following sequence analysis of any scFv to be used for display on H. They are aligned to all scFv sequences of the corresponding germline available in open access databases (e.g. Kabat or IMGT). Amino acids (AA) present in less than 10% of the germline sequences are indicated as "unusual". Next the sequences are checked for the presence of the set of AA highly stabilising scFv (as published by others), missing ones are also indicated. By comparison of unusual AA with scFv of known functionality, it is possible for the person skilled in the art to recommend changes to more stable AA in the new sequences or leave the unusual AA as they are. With this approach we achieved the following improvements for the scFv anti-CD30, anti-CD19 (clone 4G7), anti-CD8 (Okt8), anti-GluR-D (Fab22), anti-NK-cells (KYK), and anti-mCD105 (mE12):
- scFv anti-CD30: CD30-LV with the partially unstable scFv anti-CD30 form 3.4x10² t.u./175cm² VPC (vector producing cells) to 4.7x10⁵t.u./175cm² VPC
- anti-CD19 (clone 4G7): from a non-functional scFv to CD19-LV with titers of 2.7x10⁵t.u./175cm² VPC
- anti-CD8 (Okt8): from a non-functional scFv to CD8-LV with titers of 1.5x10⁵ t.u./175cm² VPC
- anti-GluR-D (Fab22): from a non-functional scFv to GluR-D-LV with titers of 1.5x10⁶ t.u./175cm² VPC
- anti-NK-cells (KYK): from a non-functional scFv to a surface expression >50%, titers were not yet determined
- anti-mCD105 (mE12): titer improvement of mCD105-LV from 7.2x10⁵ to 3.3x10⁶ t.u./175cm² VPC

We therefore claim the *in silico* method for the optimization of scFv considering the information of functionality of scFv as H-scFv fusion protein as a crucial part for development of targeted LV.

In a further embodiment the pharmaceutical compositions of the present invention are administered alone or in combination with other types of cancer treatment.

### EXAMPLES

### Materials & Methods

### Used plasmids

### Plasmids encoding envelope proteins:

**pCG-HA18** encoding the MV H gene with a truncated cytoplasmic tail of the indicated number of amino acids
**pCG-FwtA30 and pCG-FcA30** encoding the MV F gene with a truncated cytoplasmic tail of the indicated number of amino acids from Edmonston or IC332 wildtype strain.
**plasmids encoding the *gag* and *pol* genes**
**pCMVΔR8.9** encoding HIV-1 *gag* / *pol*
**Transfer plasmids**
**pSEW** is a HIV-1 packagable vector encoding the GFP reporter gene
**In vitro site-directed mutagenesis of plasmid DNA**

In vitro site directed mutagenesis allows to exchange desired nucleotides within a plasmid backbone.

For this purpose, the Stratagene Quick Change Multi Site Directed Mutagenesis Kit (Stratagene, La Jolla, USA) has been used according to the manufacturers instructions. This method is based on *Pfu* polymerase mediated extension of phosphorylated primers, which contain the desired mutation. Double stranded supercoiled plasmids served as templates and were denaturated at 95 °C for 30 seconds. Then, the temperature was lowered to 55 °C for 30 seconds to allow annealing of the primers. Subsequently, primer extension was performed at 68 °C for 12 min. Overall, 18 reaction cycles were performed. During this procedure, the newly synthesized strands were also ligated with the 5'-end of the applied primers due to heat stable ligases present in the enzyme mix. As a result, circularized DNA including the desired mutations was generated. Subsequently, the initial template strands were digested with *Dpn*I. This enzyme specifically cuts methylated DNA, as isolated from bacteria, but does not affect the newly *in vitro* synthesized strand. Thus, the reaction mixture could be used for transformation of *E. coli* cells to specifically amplify the mutated plasmids.

### Cell culture

Cells were cultivated in an incubator (Heraeus, Hanau, Germany) at 37 °C, 5 % CO₂ and saturated water atmosphere. Adherent cells were trypsinized (0.25 % trypsin in PBS) for passaging. A fraction of the resulting suspension was seeded into new culture flasks and fresh medium was added.

### used cell lines

| | |
|---|---|
| HEK-293T | DMEM with 4.5g/l glucose, 3.7 g/l NaHCO₃, 10% FCS, 1% glutamine |
| HT1080 | DMEM with 4.5g/l glucose, 3.7 g/l NaHCO₃, 10% FCS, 1% glutamine |
| HT1080-GluRD | HT1080 cells stably expressing rat GluRD |
| | DMEM with 4.5g/l glucose, 3.7 g/l NaHCO₃, 10% FCS, 1% glutamine, 3 µg/ml puromycin |
| Hut78 | RPMI 1640 with 2 g/l NaHCO₃, 10 % FCS, 1 % glutamm |
| Raji | RPMI 1640 with 2 g/l NaHCO₃, 10 % FCS, 1 % glutamm |
| Molt4.8 | RPMI 1640 with 2 g/l NaHCO₃, 10 % FCS, 1 % glutamin |

### Cell transfection

To express plasmids within eucaryotic cells either Fugene6 (Roche) or polyethyleneimine (PEI) transfection was performed.

For the qualitative detection of H-scFv fusion protein expression, 4x10⁴ HEK-293T cells (in DMEM with 4.5 g/l glucose, 3.7 g/l NaHCO₃, 10% FCS, 1% glutamine) were seeded per well in LabTekII 8-well chamberd coverslides (Nunc) 24 h before transfection. Per transfection 0.25 µg DNA was mixed with 6.25 µl OptiMEM (Gibco), then 3.75 µl of a 1:10 dilution of Fugene in Optimem was added and after 20 mm of incubation at room temperature the DNA/Fugene solution was given to the cells. Cells were then incubated for 24-48 h.

For the quantitative detection of H-scFv fusion protein expression at the cell surface, 24 h before transfection 3x10⁵ HEK-293T cells (in DMEM with 4.5 g/l glucose, 3.7 g/l NaHCO₃, 10% FCS, 1% glutamine) were seeded per well of a 24-well-plate. Per transfection 0.5 µg DNA was mixed with 100 µl OptiMEM (Gibco), then 0.75 µl Fugene was added and after 20 min of incubation at room temperature the DNA/Fugene solution was given to the cells. Cells were then incubated for 24-48 h.

For the generation of vector particles, 24 h before transfection, 2x10⁷ HEK-293T cells (in DMEM with 4.5g/l glucose, 3.7 g/l NaHCO₃, 10% FCS, 1% glutamine) were seeded into a T175 flask. Before the transfection the medium of the cells was exchanged against 7 ml DMEM with 4.5g/l glucose, 3.7 g/l NaHCO₃, 15% FCS, 1% glutamine.4,04 µg of plasmid encoding a F protein variant and 1,35 µg of plasmid encoding a H protein variant, 15,17 µg of packaging plasmid, encoding HIV-1 gag/pol, and 14,44 µg of transfer plasmid, were co-transfected by PEI transfection. 2300 µl DMEM are added to the plasmid DNA.140 µl PEI (0,78 µg/µl in H₂O (Sigma; W-3500)) are resuspended in 2200 µl DMEM, Then the plasmid and the PEI solution are combined and after 20 mm the complexed DNA is transferred to the HEK-293T cells. 15 h after this the medium is again replaced with 12 ml fresh medium.

24 h afterwards, the 12 ml cell supernatant containing the pseudotyped lentiviral vector particles, are filtered (0.45 µm filter). and concentrated by centrifugation at 100.000g (Beckman Ultracentrifuge) and 4° C for at least 16 h. The pellet was then resuspended in 120 µl serum free medium (DMEM with 4.5 g/l glucose, 3.7 g/l NaHCO₃).

### Transduction and titration

For titration of the vector particle stocks, 5x10⁴ suitable target cells were seeded into a single well of a 96-well plate. The vector stocks were serially diluted in 1:10 steps and a total of 180 ul of the dilutions, was then added to every well. After 48-72 h of incubation the titers were calculated by determining the number of green fluorescent cells per well by FACS analysis.

### Fluorescence activated cell screening (FACS)

FACS analysis allows to assay cell populations for surface expression of proteins. The method makes use of scattered light and fluorescence of labelled antibodies. In principle, FACS can also be used for intracellular stainings after membrane perfusion thus also allowing to analyse the expression of cytosolic proteins. Also the cytosolic expression of GFP can be detected.

### Detection of surface expression of H-scFv

For the quantitatve detection of surface expression of H-scFv fusion proteins 293T cells transfected with the corresponding H-scFv plasmid were detached from a 24-well-plate by incubation with 100 µl PBS-Trypsin solution. Then 1 ml FACS washing buffer (PBS, 2 % FCS, 0.1 % NaN₃) is added and this cell solution is then centrifuged for 2 mm by 3.500 rpm (Biofuge fresco Heraeus instruments) at 4°C. The pellet is resuspended in 1 ml FACS washing buffer and centrifuged as above. Then the pellet is resuspended in 100 µl FACS washing buffer and 2µl per 2x10⁵ cells of PE-labeled anti-His antibody (Miltenyi) were added. After incubation for 10 min at 4°C the cells were washed twice by adding 1 ml FACS washing buffer and centrifugation as above. Then, the cell pellet is resuspended in 200 µl PBS/1 % paraformaldehyde. Then the cell pellet is resuspended in 200 µl PBS/1 % paraformaldehyde. With the FACS machine (BD FACS LSRII) 10,000 cells are counted and the surface expression of the H-scFv fusion protein is given by the number of PE-positive cells.

### Detection of transduced cells

The transduced cells of a well with an adequate dilution are resuspended in 1 ml FACS washing buffer (PBS, 2 % FCS, 0.1 % NaN₃) and this cell solution is then centrifuged for 2 mm by 3.500 rpm (Biofuge fresco Heraeus instruments) at 4°C. The pellet is resuspended in 1 ml FACS washing buffer and centrifuged as above. Then, the cell pellet is resuspended in 200 µl PBS/1 % paraformaldehyde. With the FACS machine (BD FACS LSRII) 10,000 cells are counted and with the indicated percentage of green flourescence cells the number of transducing particles per ml can be determined. The number of cells at the timepoint of transduction is multiplied by the percentage indicated and then divided by 100. This number of transduced cells is then diveded by the µl amount of vector stock in this dilution and multiplied with 1000 µl to get the transducing units per ml (t.u./ml) or with the total amount of µl vector stock obtained from one T175 (t.u./175cm² vector packaging cells, VPC).

### Detection of surface expression via immunofluorescence staining

24h after transfection the cells were washed once with PBS, incubated for 20mm with 4% formain in PBS at room temperature, again washed anf then permeabilised with 0.05% Triton in PBS for 10min. After a next washing step with PBS, the cells were incubated with 5% chicken serum in PBS (blocking solution) for 30mm, followed by the incubation with the first antibody (anti-H, K83 1:20 dilution of crude hybridoma supernatant in blocking solution) for 1h at 37°C detecting the measles virus H protein. Then the cells were washed 3x with PBS. As secondary antibody anti-mouse IgG labeled with Cy3 (Dako; 1:250 dilution in blocking solution) was used. After incubation for 1h at 37°C the cells were washed 3 times with PBS and then kept at 4°C until analysis with the laserscan microscop (Zeiss LSM Meta).

### EXAMPLE 1:

The scFv CD30 has been cloned from a hybridoma cell line and the scFv variants obtained this way have been selected via phage display against CD30 (Hombach et al., J Immunol. Methods 1998 Sep 1;218(1-2):53-61). This scFv allowed the production of lentiviral targeting vectors (CD30-LV), however, these had only low titers. The sequences of the variable domains of the heavy (V_{H}) and light chains (V_{L}) were identified. For this purpose publicly accessible Excel-Macros were used (http://wwvv.bioc.uzh.ch/antibody/). Some of these macros were modified to address some formal issues (removal of the automatic framing function, distribution of a nucleic sequence without spaces into tripletts). The framework and CDR-regions were identified. The numbering of the amino acids in the heavy and light chains of the amino acid sequence follows Honegger und Pluckthun, J Mol Biol. 2001. CD30-V_{H} was determined to belong to murine germline V_{H} 1 (muVH1) and CD30-V_{L} to germline V-kappa 6 (muVk6). In a first step amino acids that are present in less than 90% at a given position within a germline, were identified as unusual. It was further determined whether the unusual amino acid is a similar amino acid or an amino acid with completely different properties than the amino acid that is commonly found at that position. Unusual amino acids are potentially destabilising. In a second step the sequence was examined in respect for particularly stabilising amino acids, which are described in the following publications: Ewert et al., Biochemistry. 2003 Feb 18;42(6):1517-28; Ewert et al., J Mol Biol. 2003 Jan 17;325(3):531-53; Ewert et al, Methods. 2004 Oct;34(2):184-99.; Stevens, Amyloid. 2000 Sep;7(3):200-11.; Jung et al., J Mol Biol. 1999 Nov 19;294(1):163-80; Ohage & Steipe, J Mol Biol. 1999 Sep 3;291(5):1119-28; Spada et al., J Mol Biol. 1998 Oct 23;283(2):395-407; Kipriyanov et al., Protein Eng. 1997 Apr;10(4):445-53; Lindner et al., Biotechniques. 1997 Jan;22(1):140-9; Forsberg et al., J Biol Chem. 1997 May 9;272(19):12430-6; Chan et al., Fold Des. 1996;1(2):77-89; Helms & Wetzel, J Mol Biol. 1996 Mar 22;257(1):77-86; Hurle et al., Proc Natl Acad Sci U S A. 1994 Jun 7;91 (12):5446-50. These stabilizing amino acids replace the amino acids in the germline consensus sequence. Then the following amino acids in VH: A13, H50, D51, N73, N87, N94; VL: E3, L4, F10, N20, V21, V54 and also Q5, K77, T78, T97; VL: Y23, E103 of the CD30-scFv sequence were compared to the scFv of the same germline. It was checked whether an amino acid is present in an scFv that exhibits a high surface expression and leads to high titers or in scFv that does not exhibit these features. Based on this observation it was decided whether this amino acid must be replaced or maintained. In CD30-scFv the following amino acids were exchanged via site-directed mutagenesis at the genetic level: in the VH: H50Q, D51G, N73K in the VL: E3V, L4M, F10S, N20T, V21I, Y23C and E103T. In this way the lacking disulfide bridge and the Y103-Q45 bridge in the VL has been restored and in the VH the H50, which may cause a bend in the in the sequence, and D51, a charged amino acid without defined binding partner, were exchanged against amino acids Q50 and G51 which are more common at these positions (first set of modifications). Furthermore the germline consensus was restored in V_{H} at position 73 (N73K) and in V_{L} at positions 3, 4, and 10 (E3V, L4M, F10S). Variants of scFv that carried one, two, three or all -modifications/replacements were cloned as fusion constructs with H and transfected into HEK-293T cells. After two days the H-protein expression on the surface of the cells was detected via an H-protein specific antibody using FACS and immune fluorescence. A significant increase of the H-scFv expression on the cell surface could be observed with the first set of modifications via immunofluorescence staining (Fig. 2). Also the titer of the lentiviral CD30-targeting vector with the first set of modifications showed a 300x increase, In addition, the restored germline consensus allowed even better production of lentiviral CD30-targeting vector with upto 1500-fold higher titer compared to titers reachable with the original scFv sequence (Fig. 3).

### EXAMPLE 2

scFv: cloned from published Fab-Fragment (Fab22) Jespersen et al., Eur J Biochem. 2000 Mar;267(5):1382-9,
antigen: murine glutamate receptor D
Encountered problem: no surface expression of the fusion construct H-Fab22 in the evaluation method of the invention
Sequence analysis: 7 problematic amino acids identified and replaced (V_{H}: M5Q; V_{L}: R45Q, R46Q; S75P; H88D; K92T; H97Q).
Cell surface expression of H-Fab22 determined by FACS: before replacement 5% positive cells/ after 17% positive cells
Titer of corresponding lentiviral targeting vector: before 0 t.u./after 1,5x10⁶ t.u. /175cm² VPC

### EXAMPLE 3

scFv: cloned from purchased CD8-hybridotna cell line Okt 8 (ATCC CRL-8014)
antigen: human CD8
Encountered problem: no surface expression of the fusion construct H-Okt8 in the evaluation method of the invention
Sequence analysis: problematic amino acids V_{H}: .1Q, K3Q, Q5V, E6Q, L12D, F43W, R47A, E49G, K77R, M91L, H92Q, C94S, T97R, G99E; V_{L}: K3V, F10S, A12S, P15L, E46Q, N52P, G95S, M103T, T147I, V148K, .149R
Already replacement amino acids: V_{H}: .1Q, Q5V, E6Q, L12D, F43W, E49G, K77R, C94S, T97R, G99E; V_{L}: K3V, F10S, P15L, N52P, M103T, T147I, V148K, .149R
Cell surface expression: before replacement 0%/ after 7%
Titer of corresponding lentiviral targeting vector: before 0% tu after 1,5x10⁵ t.u. /175cm² VPC

### EXAMPLE 4

scFv: derived from published scFv-fragment (KYK)
antigen: NK cell epitope
source: Kwong et al., J Mol Biol. 2008 Dec 31;384(5): 1143-56
VH: 
QVQLVESGGGLVKPGGSLRLSCAASGFTFSSYGMHWVRQAPGKGLEWVAFIRYDGSN
KYYADSVKGRFTISRDNSKNTLYLQMNSLRAEDTAVYYCAKDRGLGDGTYFDYWGQ
GTTVTVSS
VL: 
QSALTQPASVSGSPGQSITISCSGSSSNIGNNAVNWYQQLPGKAPKLLIYYDDLLPSGVS
DRFSGSKSGTSAFLAISGLQSEDEADYYCAAWDDSLNGPVFGGGTKLTVLG
Encountered problem: no surface expression of H-KYK in the evaluation method of the invention
Sequence analysis: problematic amino acids and the replacement amino acids: V_{H}: K14Q; V_{L}: A3V, A9P, S14A, S18R, I19V, K50T, F90T
Cell surface expression: before replacement 0%/ after 58%
Titer of corresponding lentiviral targeting vector: before 0 t.u./after not yet determined

### EXAMPLE 5

scFv: cloned from published scFv-fragment mE12 (Müller et al., J Immunol Methods. 2008 Nov 30;339(1):90-8)
antigen: murine endoglin (mCD105)
problem: improvement of titer of corresponding targeting vector
Problematic amino acids and their replacement amino acids: V_{H}: R17G, V53E, A98S; V_{L}: S2Y; I5T; D7P; A9S; S90T; A98P
Cell surface expression: before 48%/ after 60%
Titer of corresponding targeting vector before replacement 7x10⁵ t.u. /175cm² VPC / after: 3x10⁶ t.u. /175cm² VPC

### EXAMPLE 8

scFv: cloned from published scFv-fragment 4G7 (Peipp et al., J Immunol Methods. 2004 Feb 15;285(2):265-80)
antigen: human CD 19
VH: 
QVQLQQSGPELIKPGASVKMSCKASGYTFTSYVMHWVKQKPGQGLEWIGYINPYND
GTKYNEKFKGKATLTSDKSSSTAYMELSSLTSEDSAVYYCARGTYYYGSRVFDYWGQ
GTTLTVTVSS
VL: 
DIVMTQAAPSIPVTPGESVSISCRSSKSLLNSNGNTYLYWFLQRPGQSPQLLIYRMSNLAS
GVPDRFSGSGSGTAFTLRISRVEAEDVGVYYCMQHLEYPLTFGAGTKLEIKR
 problems no cell surface expression of the H-scFv problematic amino acids and their replacements: V_{H}: Q5V, L12D, I13V, K47R, K77R, S82V, T97R, L144., T145. ; V_{L}: A7S, A8P, P9S, I11L, P12S, P15L, L45Q, A88D, A98P, V103T
cell surface expression: before 0.4% / after: 50%
Titer of corresponding targeting vector before replacement 0 t.u. /175cm² VPC / after: 2.7x10⁵ t.u. /175cm² VPC

### REFERENCES CITED

Altschul, S.F., Gish, W., Miller, W., Myers, E.W. & Lipman, D.J., J. Mol. Biol. 215:403-410 (1990).
Cattaneo and Rose, 1993; J Virol 67, 1493-1502.
Cathomen T., et al., Virology 214:628-632 (1995).
Engelstaedter et al., 2000; Human Gene Therapy 11, 293.
Griffiths et al., 1994; EMBO J., 13, 3245-3260.
Laemmli UK, "Cleavage of structural proteins during the assembly of the head of bacteriophage T4," Nature 227:680-685 (1970).
Masse et al. (2002) J Virol 76 (24) p. 13034-13038.
Masse et al. (2004) J Virol 78 (17) p. 9051-9063.
Nakamura et al. (2004) Nat. Biotech. 22 (3) p. 331-336.
Nakamura et al. (2005) Nat. Biotech. 23 (2) p.209-214.
Patterson et al. (1999) Virology 256 p. 142-151.
Pearson WR and Lipman DJ, "Improved tools for biological sequence comparison", Proc Natl Acad Sci USA 85:2444-2448 (1988).
Plemper RK, Hammond AL, Cattaneo R., "Measles virus envelope glycoproteins heterooligomerize in the endoplasmic reticulum", J Biol Chem. 2001, 23;276(47):44239-46.
Saiki RK, Gelfand DH, Stoffel S, Scharf SJ, Higuchi R, Horn GT, Mullis KB, and Erlich HA, Science 239:487 (1988).
Sambrook J, Fritsch EF, and Maniatis T, Molecular Cloning. A Laboratory Manual, 2d ed., Cold Spring Harbor Laboratory, Cold Spring Harbor (1989).
Toennes et al., J. Neurochem. 73, 2195 (1999).
Xu K, Ma H, McCown TJ, Verma IM, Kafri T., Mol. Ther. 3:97-104 (2001).
Vongpunsawad et al. (2004) J Virol 78 (1) p. 302-313.
Zoller MJ, and Smith M, Nucleic Acids Res 10:6487-6500 (1982).
Zoller MJ, Methods Enzymol 100:468-500 (1983).
Zoller MJ, DNA 3(6):479-488 (1984).

## Claims

1. A method for evaluating the stability of an scFv, comprising the steps:
a) expressing in a mammalian cell line a fusion protein comprising N-terminally a first amino acid sequence comprising a mammalian cellular export signal, and C-terminally a second amino acid sequence comprising the amino acid sequence of an scFv;
b) determining whether the fusion protein is exported from the inside of the cell to the outside of the cell; and
c) designating scFvs comprised in fusion proteins that are exported from the inside of the cell to the outside of the cell as stable, and designating scFvs comprised in fusion proteins that are not exported from the inside of the cell to the outside of the cell as unstable.

2. The method of claim 1 for optimizing the stability of an scFv, further comprising the steps:
d) determining the germline of an scFv analyzing the amino acid sequences of a first group of scFvs designated as stable in step c);
e) identifying in the amino acid sequences of the scFvs analyzed in step d) an amino acid that is located at a given position in the amino acid sequence in at least 75% of the amino acid sequences;
f) replacing in an amino acid sequence of an scFv designated as unstable in step c) the amino acid located at the given position in the amino acid sequence with the amino acid identified in step e) to thereby obtain an optimized fusion protein; and
g) determining whether the fusion protein obtained in step f) is exported from the inside of the cell to the outside of the cell,
wherein the steps e) - g) can be repeated.

3. The method according to claim 1 or 2, wherein the first amino acid sequence corresponds to the measles H-protein, optionally wherein the H protein is recombinant or mutated.

4. The method according to any of claims 1 to 3, wherein the wherein the mammalian cell line is HEK-293T.

5. A single-chain variable fragment (scFv) comprising an immunoglobulin heavy chain variable region (V_{H}) and an immunoglobulin light chain kappa variable region (V_{LK}), wherein the V_{H} comprises Ala at position 25, Phe or Tyr at position 29, Ser or Lys at position 86, and Ala or Leu at position 89, the V_{LK} comprises Gln at position 46, Thr at position 87 and Gln at position 97, and wherein the positions are designated as published by Honegger and Plückthun (JMB (2001) 309:957-670).

6. The scFv of claim 5, wherein the V_{LK} further comprises at least one amino acid selected from the group consisting of Asp at position 1, Lys at position 47, Leu at position 55, Ser at position 79, Gly at position 82, and Phe at position 89.

7. The scFv according to any of claims 5-6, wherein the scFv is fused to the ectodomain of a hemagglutinin (H)-protein, optionally wherein the scFv protein is C-terminally fused to the H protein.

8. The scFv of claim 7, wherein the H-protein is derived from a virus, optionally wherein the virus is selected from the group of paramyxovirus, e.g. measles virus.

9. The scFv according to claim 7 or 8, wherein the H protein is recombinant and/or wherein the H protein is mutated.

10. The scFv of any of claims 7 to 9 claims for use in H-scFv display.

11. The scFv of claims 5 to 9 for use in a cancer test system.

12. The scFv of claims 5 to 9 for use in the treatment of cancer, optionally wherein the cancer is selected from the group consisting of leukemia, lymphoma, fibrosarcoma, myxosarcoma, liposarcoma, chondrosarcoma, osteogenic sarcoma, angiosarcoma, endotheliosarcoma, Ewing's tumor, colon carcinoma, pancreatic cancer, breast cancer, ovarian cancer, prostate cancer, squamous cell carcinoma, basal cell carcinoma, adenocarcinoma, renal cell carcinoma, hepatoma, Wilms' tumor, cervical cancer, uterine cancer, testicular tumor, lung carcinoma, small cell lung carcinoma, bladder carcinoma, epithelial carcinoma, glioma, astrocytoma, oligodendroglioma, melanoma, neuroblastoma, retinoblastoma, dysplasia and hyperplasia. In a particular embodiment, therapeutic compounds of the invention are administered to patients having prostate cancer (e.g., prostatitis, benign prostatic hypertrophy, benign prostatic hyperplasia (BPH), prostatic paraganglioma, prostate adenocarcinoma, prostatic intraepithelial neoplasia, prostato-rectal fistulas, and atypical prostatic stromal lesions). The treatment and/or prevention of cancer includes, but is not limited to, alleviating symptoms associated with cancer, the inhibition of the progression of cancer, the promotion of the regression of cancer, and the promotion of the immune response..

13. An isolated host cell comprising the scFv of any of claims 5-9.

14. The isolated host cell of claim 13, wherein the host cell is an isolated mammalian cell, preferably wherein the host cell is human cell, optionally wherein the host cell is selected from the group consisting of HEK-293T.

15. The isolated host cell of any of claims 13 or 14, wherein the host cell is a hybridoma.
